# EUROPEAN PATENT APPLICATION

(11) **EP 4 458 326 A1**
(43) Date of publication of application: **06.11.2024**
(21) Application number: 22914158.5
(22) Date of filing: 09.12.2022
(51) Int. Cl.: A61F 2/88

(54) **ENDOLUMINAL STENT**

(30) Priority: 31.12.2021 CN 202111677226; 31.12.2021 CN 202111677241
(71) Applicant: Lifetech Scientific (Shenzhen) Co., Ltd., Shenzhen, Guangdong 518063 (CN)
(72) Inventor: XIAO, Benhao, Shenzhen, Guangdong 518063 (CN); MING, Tingbo, Shenzhen, Guangdong 518063 (CN); YANG, Wulong, Shenzhen, Guangdong 518063 (CN); YAN, Jin, Shenzhen, Guangdong 518063 (CN)
(74) Representative: Michalski Hüttermann & Partner Patentanwälte mbB
(86) International application number: PCT/CN2022/137946
(87) International publication number: WO 2023/124903

(57) **Abstract**

A lumen stent including a main body stent and a branch stent; the main body stent includes a tubular attachment segment, the attachment segment including a first region and a second region connected to the first region in a circumferential direction; the branch stent is provided in a lumen of the attachment segment and connected to the first region; an axial shortening rate of the first region is smaller than an axial shortening rate of the second region. The lumen stent can reduce the extent to which the branch stent drives the main body stent to shorten.

## Description

### Technical Field

The embodiments relate to the field of medical devices and to a lumen stent.

### Background

At present, microtrauma intervention can be used to treat vascular diseases. Such methods have little trauma to patients, high safety, and high effectiveness, so it has been affirmed by doctors and patients and have become an important treatment method for vascular diseases. Interventional therapy refers to the use of a delivery system to implant vascular stents into the patient's vascular lesion segment. The implanted vascular stents can support the narrow occlusive segment of blood vessels or block the vascular dissection rupture through expansion, reduce the elastic retraction and remodeling of blood vessels, keep the lumen blood flow unobstructed, and prevent restenosis.

For the endovascular treatment of aortic dissection or aortic aneurysm involving branch arteries, bridging stent technology has become a trend in the treatment of aortic dissection or aortic aneurysm involving branch arteries because of its advantages such as low postoperative internal leakage, good stent integrity, and continuous blood supply from branch arteries during implantation. Bridging stent technology can be divided into branch stent technology and external bridging stent technology according to the position relationship between bridging stent and main body stent. Among them, branch stent technology has the advantages of easy assembly and relatively low surgical difficulty, and has become a significant area in current research.

The existing branch stents are fixed to the inner wall of the main body stent by sutures, for example, by stitching from one end of the branch stent to the other end thereof through the sutures, ensuring that the entire branch stent is closely attached to the inner wall of the main body stent. After the stent is implanted in the target position, some parts of the conveyor (for example, Tip heads) may hook the branch stent during the process of withdrawing the conveyor from the near end to the far end, causing the branch stent drives the main body stent to shorten.

### Summary

The embodiments provide a lumen stent, the object of which is to reduce the extent to which the branch stent drives the main body stent to shorten. The object is achieved through the following solutions.

The embodiments provide a lumen stent including a main body stent and a branch stent; the main body stent includes a tubular attachment segment, the attachment segment including a first region and a second region connected to the first region in a circumferential direction; the branch stent is provided in a lumen of the attachment segment and connected to the first region; an axial shortening rate of the first region is smaller than an axial shortening rate of the second region.

In one embodiment, the first region includes a plurality of first waveform units arranged axially, and the second region includes a plurality of second waveform units arranged axially, a shortest axial distance between two adjacent first waveform units being less than a shortest axial distance between two adjacent second waveform units.

In one embodiment, a wave height of the first waveform unit is greater than a wave height of the second waveform unit.

In one embodiment, the first region includes adjacent proximal first waveform units and distal first waveform units; the proximal first waveform units include first proximal valleys, and the distal first waveform units include first distal peaks; at least one of the first proximal valleys is opposite to one of the first distal peaks, and a line between the first distal peak and the first proximal valley is substantially parallel to an axis of the main body stent.

In one embodiment, the second region includes a proximal second waveform unit and a distal second waveform unit; the proximal second waveform unit includes a plurality of second proximal valleys located in different radial planes, and the distal second waveform unit includes a plurality of second distal peaks located in different radial planes; the second proximal valley circumferentially closer to the first region is closer to a proximal end of the main body stent, and/or the second distal peak circumferentially closer to the first region is closer to a distal end of the main body stent.

In one embodiment, a wave height of the first waveform unit is substantially equal to a wave height of the second waveform unit; phases of adjacent first waveform units are opposite, and phases of adj acent second waveform units are the same or have a phase difference.

In one embodiment, the first region includes a plurality of first waveform units arranged axially, and at least one first elastic member is provided between adjacent first waveform units.

In one embodiment, the branch stent has a proximal port movable relative to the main body stent.

In one embodiment, the lumen stent further includes a second elastic member; the second elastic member is disposed between an inner surface of the main body stent and an inner surface of the branch stent; the second elastic member is elastic and is used to limit a range of motion of the proximal port of the branch stent.

In one embodiment, at least one of the branch stents is provided with a linear or curved support wire, one end of the support wire being closer to a proximal end of the branch stent than the other end.

In one embodiment, the support wire is located on an outer surface or inner surface of the branch stent.

In one embodiment, the proximal end of the support wire passes over the proximal port of the branch stent.

In one embodiment, the main body stent includes a main body covering film and at least one main body wave loop disposed within the main body covering film; the proximal end of the support wire and the main body wave loop about each other when the lumen stent is in a naturally unfolded state.

In one embodiment, the main body stent includes an anchoring portion;
the branch stent provided within a lumen of the main body stent and includes a free end movable relative to the main body stent; and
a connector, connecting the free end of the branch stent and the anchoring portion.

In one embodiment, the main body stent includes a covering film and a plurality of axially spaced main body wave loops connected to the covering film; the anchoring portion includes a bare wave loop and/or an anchor, a radial support force of the bare wave loop being greater than a radial support force of the main body wave loop.

In one embodiment, the free end of the branch stent is located at a proximal end of the branch stent, and the anchoring portion is located at a proximal end of the main body stent; or, the free end of the branch stent is located at a distal end of the branch stent, and the anchoring portion is located at a distal end of the main body stent.

In one embodiment, the connector includes a linear or curved support rod; one end of the support rod is connected to the anchoring portion, and the other end of the support rod is connected to the free end of the branch stent.

In one embodiment, the connector includes an elastic segment, two ends of the elastic segment connecting to the anchoring portion, and the free end of the branch stent, respectively; the elastic segment includes one or more of a helical spring structure, a planar folding structure, and an elastomer member.

In one embodiment, the planar folding structure includes a sawtooth wave; the sawtooth wave includes a sawtooth tilted toward the anchoring portion or the branch stent.

In one embodiment, the connector includes a helical segment; the helical segment extends helically entirely around an axis of the main body stent; and the helical segment at least partially conforms to and is movable relative to an inner surface of the main body stent.

According to the lumen stent of the embodiments, because the branch stent is connected to the first region with a low axial shortening rate, the first region connected to the branch stent can greatly reduce the degree of shortening of the main body stent even if the main body stent is carried by the branch stent.

### Brief Description of the Drawings

Various other advantages and benefits will become apparent to the ordinarily skilled in the art upon reading the following detailed description of the implementations. The drawings are only for the purposes of illustrating the implementations and are not to be construed as limiting the embodiments.
Fig. 1 is a structural diagram of a lumen stent according to an embodiment;
Fig. 2 is a top view of an attachment segment in an embodiment;
Fig. 3 is a front view of an attachment segment in an embodiment;
Fig. 4 is an expanded view of a pair of main body wave loops of an attachment segment of Fig. 2;
Fig. 5 is an expanded view of a pair of main body wave loops of an attachment segment according to another embodiment;
Fig. 6 is a structural diagram of a branch stent according to an embodiment;
Fig. 7 is a side view of a transition segment of a branch stent of Fig. 6;
Fig. 8 is a front view of an attachment segment in another embodiment;
Fig. 9 is an expanded view of a pair of main body wave loops of an attachment segment of Fig. 8;
Fig. 10 is an expanded view of a pair of main body wave loops of an attachment segment according to another embodiment;
Fig. 11 is a structural diagram of a branch stent according to another embodiment;
Fig. 12 is a structural diagram of a support wire of a branch stent in another embodiment;
Fig. 13 is a structural diagram of a first elastic member according to an embodiment;
Fig. 14 is a structural diagram of a first elastic member according to another embodiment;
Fig. 15 is a coupling position diagram of a second elastic member according to an embodiment;
Fig. 16 is a structural diagram of a second elastic member of Fig. 15;
Fig. 17 is a coupling position diagram of a second elastic member according to another embodiment;
Fig. 18 is a coupling position diagram of a second elastic member according to another embodiment;
Fig. 19 is a structural diagram of a second elastic member according to another embodiment;
Fig. 20 is a structural diagram of a second elastic member according to still another embodiment;
Fig. 21 is a structural diagram of a lumen stent according to an embodiment;
Fig. 22 is a structural diagram of a lumen stent according to another embodiment;
Fig. 23 is a structural diagram of a connector of Fig. 22;
Figs. 24a and 24b are structural diagrams of a connector according to another embodiment;
Fig. 25 is a structural diagram of a connector according to another embodiment;
Fig. 26 is a structural diagram of a connector having an intermediate member according to an embodiment;
Fig. 27 is a structural diagram of a connector having an intermediate member according to another embodiment;
Fig. 28 is a structural diagram of a connector having a helical segment according to an embodiment; and
Fig. 29 is a structural diagram of a connector having a helical segment according to another embodiment.

### Detailed Description of the Embodiments

Exemplary embodiments will be described in more detail below concerning the accompanying drawings. While the exemplary implementations of the embodiments are shown in the drawings, the embodiments can be implemented in various forms and should not be construed as limited to the implementations set forth herein. These implementations are provided so that the descriptions will be thorough and complete, and will fully convey the concept of the embodiments to those skilled in the art.

It is to be understood that the terminology used herein is to describe specific example implementations only and is not intended to be limiting. As used herein, the singular forms "a", "an", and "the" are intended to include the plural forms as well, unless the context indicates otherwise. The terms "comprise", "include", "contain", and "have" are inclusive and therefore specify the presence of stated features, steps, operations, elements, and/or components, but do not preclude the presence or addition of one or more other features, steps, operations, elements, components, and/or groups thereof. The method steps, processes, and operations described herein are not to be construed as necessarily requiring that they be performed in the specific order described or illustrated unless an order of performance is explicitly stated. It should also be understood that additional or alternative steps may be used.

Although the terms first, second, third, and the like may be used herein to describe various elements, components, regions, layers, and/or sections, these elements, components, regions, layers, and/or sections should not be limited by these terms. These terms may only be used to distinguish one element, component, region, layer, or section from another region, layer, or section. The use of terms such as "first", "second", and other numerical terms herein do not imply a sequence or order unless the context dictates otherwise. Thus, a first element, component, region, layer, or section discussed below could be termed a second element, component, region, layer, or section without departing from the teachings of the example implementations.

For ease of description, spatially relative terms, such as "inner", "outer", "inside", "outside", "below", "under", "above", "over", and the like, may be used herein to describe one element or feature's relationship to another element(s) or feature(s) as illustrated in the drawings. Such spatially relative terms are intended to encompass different orientations of the device in use or operation in addition to the orientation depicted in the drawings. For example, if the device in the drawings is turned over, elements described as "below" or "under" other elements or features would then be oriented "above" or "over" the other elements or features. Thus, the exemplary term "below" or "under" can include both an upper and a lower orientation. The device may be otherwise oriented (rotated 90 degrees or in other directions) and the spatially relative descriptors used herein interpreted accordingly.

To describe the structure of the embodiments more clearly, the terms "proximal" and "distal" are defined herein as conventional terms used in the art of interventional medicine. For example, "distal" refers to the end from which blood flows, and "proximal" refers to the end into which blood flows, for example, after stent implantation, blood flows from the proximal end toward the distal end of the stent; "axial" refers to its lengthwise direction, and "radial" refers to a direction perpendicular to the "axial" direction.

A "wave loop" in the embodiments is a closed ring structure, also known as a wavy ring, woven, or cut from a metal elastic material. The metallic elastic materials include known materials in implantable medical devices or combinations of various biocompatible materials, such as alloys of two or more single metals of cobalt, chromium, nickel, titanium, magnesium, iron, as well as 316L stainless steel, nickel-titanium-tantalum, or other biocompatible metallic elastic materials.

The wave loop has a radial expansion capacity and can achieve radial contraction under the action of external force, and recover to the original shape by self-expansion or mechanical expansion (for example, expansion through balloon expansion) after the external force is withdrawn, and maintains the original shape so that it can abut against the inner wall of lumen through its radial supporting force after the lumen is implanted. The waveform of the wave loop is not limited and includes a Z-shaped wave, an M-shaped wave, a V-shaped wave, a sine wave, and the like. The wave loop includes a plurality of peaks (also referred to as proximal vertices), a plurality of valleys (also referred to as distal vertices), and a rod connecting adjacent peaks and valleys. One vertex (proximal vertex or distal vertex) and two rods connected to the vertex form one wave.

As used herein, "wave number" means the number of peaks or valleys, and the number of peaks and valleys in the same wave loop are the same. As used herein, "wave height" refers to the perpendicular distance between a peak and two adjacent valleys.

About the shortest axial distance between adjacent waveform units as referred to in the embodiments, two axially adjacent waveform units, such as waveform unit A and waveform unit B, are described herein as an example, waveform unit A is located at the proximal end of waveform unit B, and if waveform unit A is to abut waveform unit B, the shortest axial distance required to move is the shortest axial distance.

### Embodiment 1

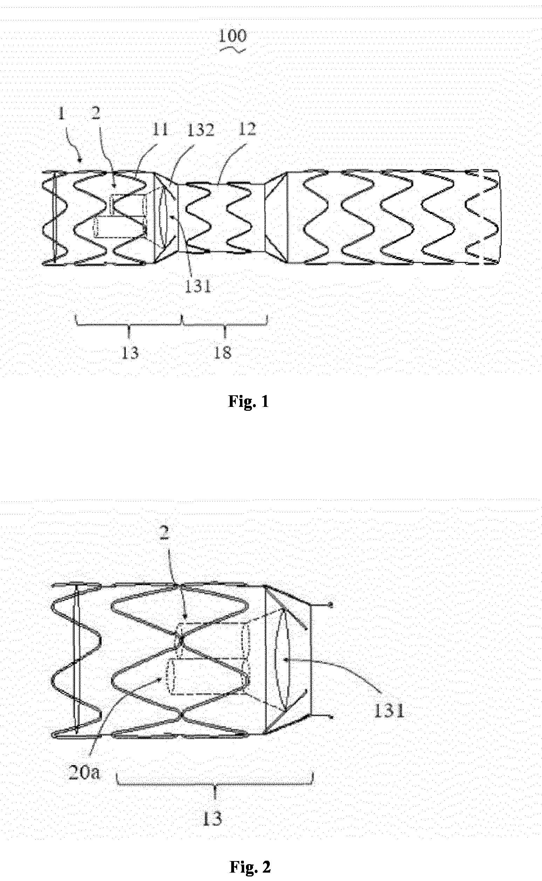

As shown in Figs. 1 to 3, the lumen stent 100 of the embodiment includes a main body stent 1 and a branch stent 2.

The main body stent 1 is a tubular structure having openings at both ends, and includes a main body wave loop 11 and a main body covering film 12, and a plurality of main body wave loops 11 are arranged axially and connected by the tubular main body covering film 12.

The main body stent 1 includes at least one attachment segment 13, the attachment segment 13 is tubular, and the branch stent 2 is provided in the lumen of the attachment segment 13. In the embodiment, in the attachment segment 13, the main body wave loop 11 is provided on the outer side of the main body covering film 12 to enhance the frictional force between the attachment segment 13 and the inner wall of the lumen to help prevent the main body stent 1 from shifting and shortening relative to the lumen. In other embodiments, the main body wave loop 11 may be provided inside the main body covering film 12, or the main body covering film 12 may be provided both inside and outside the main body wave loop 11.

In the embodiment, the cross-sectional shape of the attachment segment 13 is generally circular to better conform to the inner wall of the lumen. In other embodiments, the cross-sectional shape of the attachment segment 13 may be any other suitable shape, such as elliptical.

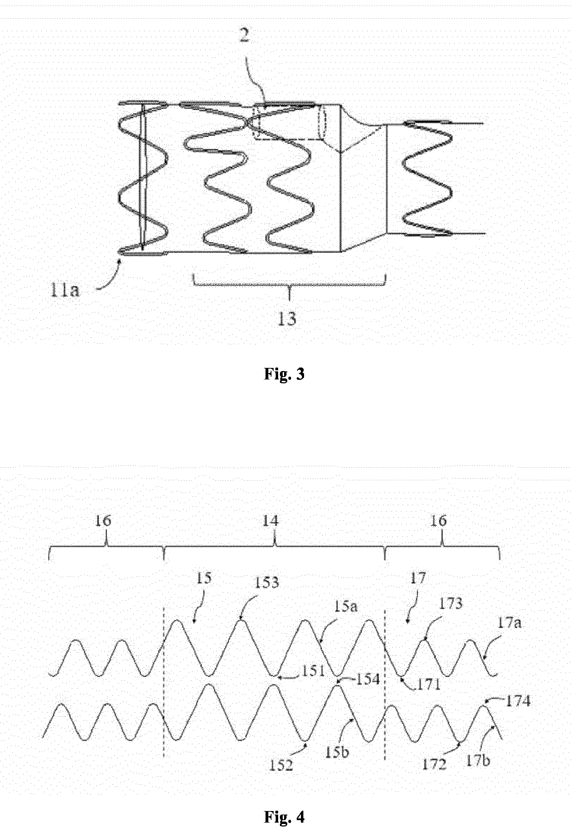

Referring to Fig. 4, in the embodiment, the attachment segment 13 includes a first region 14 and a second region 16 connected to the first region 14 in the circumferential direction. The first region 14 includes a plurality of first waveform units 15 arranged axially. The second region 16 includes a plurality of second waveform units 17 arranged axially. Both the first waveform unit 15 and the second waveform unit 17 are part of the main body wave loop 11. In the embodiment, the first region 14 includes two first waveform units 15 arranged axially, a proximal first waveform unit 15a closer to the proximal end, and a distal first waveform unit 15b closer to the distal end. The second region 16 also includes two second waveform units 17, a proximal second waveform unit 17a closer to the proximal end and a distal second waveform unit 17b closer to the distal end.

The first waveform unit 15 includes a plurality of high waves whose wave heights are substantially uniform. The second waveform unit 17 includes a plurality of short waves of substantially uniform wave height. The ratio of the wave height of the first waveform unit 15 to the wave height of the second waveform unit 17 is 1.2 to 1.8.

The proximal first waveform unit 15a includes a first proximal valley 151 and a first proximal peak 153, and the distal first waveform unit 15b includes a first distal valley 152 and a first distal peak 154. The proximal second waveform unit 17a includes a second proximal valley 171 and a second proximal peak 173, and the distal second waveform unit 17b includes a second distal valley 172 and a second distal peak 174.

The proximal first waveform unit 15a and the distal first waveform unit 15b are opposite in phase, i.e. the first proximal valley 151 is opposite (close to each other) to the first distal peak 154, and the first proximal peak 153 is separated (far from each other) from the first distal valley 152, at least one line connecting the first proximal valley 151 and the first distal peak 154 is substantially parallel to the axis of the main body stent 1.

The proximal second waveform unit 17a and the distal second waveform unit 17b are opposite in phase, that is, the second proximal valley 171 is opposite to the second distal peak 174, and the second proximal peak 173 is separated from the second distal valley 172, and at least one line connecting the second proximal valley 171 and the second distal peak 174 is substantially parallel to the axis of the main body stent 1. Alternatively, the proximal second waveform unit 17a and the distal second waveform unit 17b are out of phase, i.e. the second proximal valley 171 is opposite to the second distal peak 174, and any line connecting the second proximal valley 171 and the second distal peak 174 is at an angle to the axis of the main body stent 1.

Referring to Fig. 4, in the embodiment, the first proximal valley 151 and the second proximal valley 171 are substantially located in the same radial plane, and the first distal valley 152 and the second distal valley 172 are also substantially located in the same radial plane.

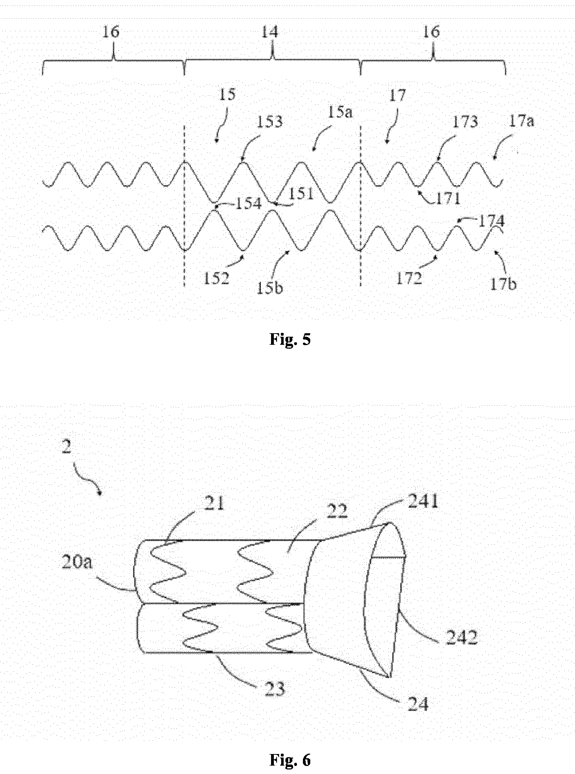

Referring to Fig. 5, in other embodiments, the first proximal peak 153 and the second proximal peak 173 are substantially located in the same radial plane, and the first distal valley 152 and second distal valley 172 are also substantially located in the same radial plane. Such an arrangement is beneficial to increase the axial distance between adjacent second waveform units 17 so that the second region 16 is more easily bent, and when the second region 16 is implanted to the side of a small curve of the aortic arch (such as the side with a larger bending degree and a smaller curvature radius), the bending shape of the side of the small curve can be more closely fitted, thereby increasing the friction force between the main body stent 1 and the inner wall of the lumen, and further preventing shortening; in addition, in Fig. 5, both the proximal first waveform unit 15 and the distal first waveform unit 15b can be made by the same die (or tooling), and the proximal second waveform unit 17a and the distal second waveform unit 17b can also be made by the same die (or tooling), so that the manufacturing process is simpler and more efficient.

In the embodiment, the shortest axial distance (also referred to as axial spacing) between two adjacent first waveform units 15 is smaller than the shortest axial distance between two adjacent second waveform units 17. For example, the shortest axial distance between adjacent first waveform units 15 is 0 to 2 mm. In other embodiments, where the first region 14 includes more than two first waveform units 15 and the second region 16 includes more than two second waveform units 17, the shortest axial distance between any two adjacent first waveform units 15 is less than the shortest axial distance between any two adjacent second waveform units 17.

Because the shortest axial distance between two adjacent first waveform units 15 in the first region 14 is smaller than the shortest axial distance between adjacent second waveform units 17, and the wave height of the first waveform unit 15 is larger than the wave height of the second waveform unit 17, therefore, when shortening occurs under the action of an external force, the amount by which the first region 14 can be shortened in the axial direction is smaller than the amount by which the second region 16 can be shortened in the axial direction. In addition, in the embodiment, the axial lengths of the first region 14 and the second region 16 are the same, so the axial shortening rate of the first region 14 is smaller than that of the second region 16. The axial shortening rate refers to the ratio of the axial shortening length to the axial length in the natural unfolded state when the axial compression force F (for example, 1N ≤ F ≤ 2N) is applied to make the axial shortening no longer possible in the natural unfolded state. The axial shortening of the first zone 14 and the second zone 16 can be measured by the following method: in a naturally unfolded state, the length of the region to be measured (the first region 14 or the second region 16) is a and the diameter is d, the attachment segment 13 is sleeved in an inner tube (for example, a relatively smooth inner tube) with a diameter of 0.9d, an axial pressure is uniformly applied to both ends of the region to be measured (for example, 1 N to 2 N), once there is a position where the region to be measured cannot be shortened any more, the axial pressure is stopped, and the axial length of the region to be measured is b, and the axial shortening rate of the region to be measured is (a-b) ÷ a × 100%.

It is to be noted that the shortest axial distance between the above-mentioned adjacent first waveform units 15 should be appropriate, and when the shortest axial distance is too long so that the axial shortening rate of the first region 14 is too large, and when the shortest axial distance is too short so that the first waveform units 15 are extended toward both ends due to radial compression, the opposite peaks and valleys of the adjacent first waveform units 15 are easily stacked together, thereby increasing the difficulty in the assembly of the lumen stent 100 and increasing the assembly force.

The surface of the attachment segment 13 is provided with a window 131, and the window 131 is opened at the position of no wave loop on the main body covering film 12. For example, the surface of the main body stent 1 may form a recessed segment 18, the attachment segment 13 may be provided with a ramp 132 connected to the recessed segment 18, the ramp 132 being formed by a portion of the main body covering film 12, and the ramp 132 may be provided with the window 131. The recessed segment 18 can provide a larger extension space for the bridge stent at the junction of the bridge stent and the main body stent 1 at the time of implantation of the bridge stent (not shown), so that the bridge stent is not easily occluded at the time of implantation and after implantation.

The branch stent 2 is provided in the lumen of the attachment segment 13, and the lumen of the branch stent 2 communicates with the window 131, to ensure that the implanted bridging stent can be smoothly inserted into the branch stent 2 through the window 131. In other embodiments, the main body stent 1 may omit the window, the lumen of the branch stent 2 communicates with the lumen of the main body stent 1, and the bridging stent may enter the main body stent 1 from the distal end of the main body stent 1 and plug onto the branch stent 2.

Referring to Fig. 6, the branch stent 2 includes a branch wave loop 21 and a branch covering film 22, and a plurality of branch wave loops 21 are connected by the branch covering film 22. The branch stent 2 includes two branch tubes 23 and a transition segment 24. The distal end of the transition segment 24 is connected to window 131 in a sealing manner (see Fig. 1), and the distal ends of the two branch tubes 23 are connected to the proximal end of the transition segment 24, and the proximal end of the branch tube 23 is a free end and is arranged towards the proximal end of the main body stent 1. The branch tube 23 and the transition segment 24 may be of a one-piece structure formed by arranging a plurality of branch wave loops 21 at intervals and laminating the inner and/or outer sides thereof. In the naturally unfolded state, the axis of the branch tube body 23 is substantially parallel to the axis of the main body stent 1, which can reduce the difficulty of the bridging stent implantation to a certain extent without greatly affecting the blood flow in the lumen of the main body stent 1. In other embodiments, the angle between the axial direction of the branch tube 23 and the axial direction of the main body stent 1 may be any other angle in the range of 0° to 15°. In addition, the number of branch tubes 23 is not limited, and can be one or more than two, and can be set and selected according to the number and position of bridging stents to be accessed, so that it can meet the needs of emergency surgery in clinical use, and can avoid missing the optimal time of treatment due to long waiting time for customization. In addition, the branch covering film 22 on the branch stent 2 may be formed by joining a plurality of pieces of the covering film by sewing, bonding, or the like.

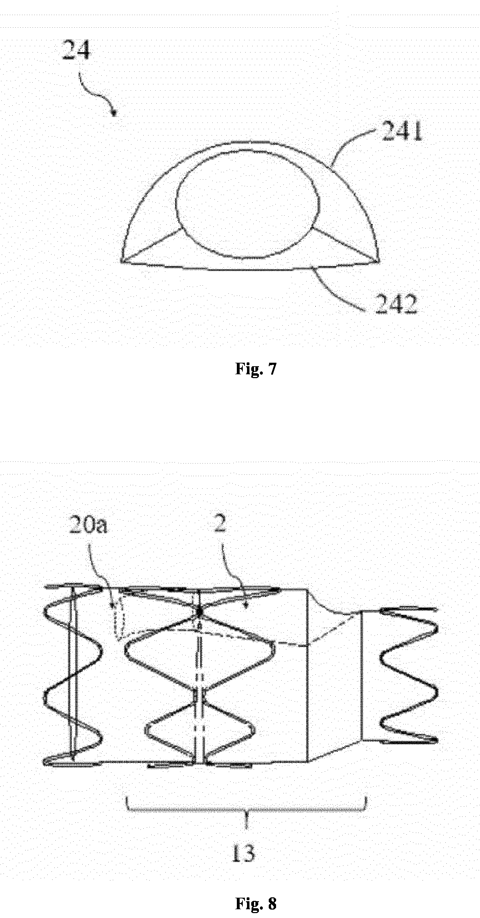

Referring also to Fig. 7, the transition segment 24 includes an arcuate sheet 241 and a relatively flat bottom sheet 242, the arcuate sheet 241 and the bottom sheet 242 enclosing a tubular structure having a generally crescent-shaped cross-section, the arcuate sheet 241 being disposed closer to the inner wall of the attachment segment 13 than the bottom sheet 242 to provide a better fit between the transition segment 24 and the inner wall of the attachment segment 13. In addition, the transition segment 24 includes a small diameter end connected to the distal end of the branch tube 23 and a large diameter end connected to the window 131. The radial dimension of the small diameter end matches the radial dimension of the branch tube 23, and the radial dimension of the large diameter end matches the dimension of the window 131, to achieve a sealed connection between the small diameter end and the branch tube 23, and a sealed connection between the transition segment 24 and the window 131. The transition segment 24 and the window 131 and the branch tube 23 can be connected by sewing, bonding, and the like. The edge of the large diameter end of the transition segment 24 or the edge of the window 131 may also be provided with an annular support to keep the morphology of the window 131 stable.

The shape of the window 131 of the embodiments is not limited, and may be circular, elliptical, crescent-shaped, or any other shape, with the radial dimension of the window 131 being larger than the radial dimension (for example, the diameter) of the branch tube 23 to facilitate implantation of a bridging stent. In other embodiments, a plurality of windows 131 and a plurality of branch stents 2 may also be provided on the surface of the main body stent 1, the plurality of windows 131 is arranged at intervals along the axial direction of the main body stent 1, so that the specific fixing position of the main body stent 1 can be adjusted according to the structure of the arterial vessel, thereby performing the reconstruction of the blood supply of the plurality of branch vessels. It will be understood that in the embodiment, the branch tube 23 of the branch stent 2 is closer to the proximal end of the main body stent 1 than the transition segment 24, and in other embodiments, the branch tube 23 of the branch stent 2 may be closer to the distal end of the main body stent 1 than the transition segment 24, or when a plurality of branch stents 2 are provided, in the plurality of branch stents 2, the branch tube 23 of one part of the branch stent 2 is closer to the proximal end of the main body stent 1 and the main body of the other part of the branch stent 2 is closer to the distal end of the main body stent 1.

Referring also to Figs. 1 to 3, in the embodiment, the branch stent 2 is connected to the first region 14, and the distance of the branch stent 2 from the first region 14 is less than the distance of the branch stent 2 from the second region 16. Since the branch stent 2 is connected to the first region 14 having a low axial shortening rate, even if the branch stent 2 is caught by the conveyor and causes the main body stent 1 to deform, the first region 14 connected to the branch stent 2 can greatly reduce the shortening rate of the main body stent 1.

In the embodiment, the branch stent 2 is attached to the inner wall of the first region 14, and the tangent regions of the branch stent 2 and the first region 14 are secured to each other by stitching. For example, a tangent line extending substantially in the axial direction is provided between the branch tube 23 and the inner wall of the first region 14, a suture is used to suture and fix the branch tube 23 and the first region 14 at the tangent point (such as along the tangent line) between the branch tube 23 and the inner wall of the first region 14, so that a plurality of fixing points are formed on the tangent line of the branch tube 23 and the inner wall of the first region 14; fixing points are provided on both the proximal port (such as the proximal port 20a of the branch stent 2) and the distal end port of the branch tube 23, to ensure that the shape of the branch stent 2 does not change greatly under the impact of blood flow or when being hooked by the conveyor, effectively prevent the shortening of branch stent 2. In addition, the side of the transition segment 24 that conforms to the first region 14 (i.e. the arcuate sheet 241) may be connected to the first region 14 with sutures to provide a better fit between the transition segment 24 and the inner wall of the first region 14 and to minimize the gap between the transition segment 24 and the first region 14 to avoid blood pooling in the gap and thrombosis. In other embodiments, only one end of the transition segment 24 is connected to the window 131, the other end is connected to the branch tube 23, and other regions may not be fixed to the first region 14. Further, in other embodiments, the branch stent 2 may be fixedly connected to the first region 14 by bonding or the like.

Referring also to Figs. 1 and 4, the length and location of the branch tubes 23 affect performance. In the embodiment, the ratio of the length of the branch tube 23 to the wave height of the first waveform unit 15 maybe 0.5 to 2.2, and the proximal port of the branch tube 23 is substantially located in the same radial plane as the crest of the proximal first waveform unit 15a, or the proximal port of the branch tube 23 is located between the first proximal crest 153 and the first distal valley 152. The purpose of this arrangement is to position the branch tube 23 in a position where the first waveform unit 15 is supported in the first region 14, thereby making it more difficult for the branch stent 2 to cause the main body stent 1 to shorten.

Referring to Fig. 3, in the embodiment, the most proximal main body wave loop 11 of the main body stent 1 is a bare wave loop 11a, and a plurality of valleys of the bare wave loop 11a are fixedly connected to the inner side of the proximal edge of the main body covering film 12, and a part of the bare wave loop 11a is exposed, that is, the main body covering film 12 only partially covers the bare wave loop 11a. On the one hand, the bare wave loop 11a can improve the anchoring force between the main body stent 1 and the lumen, on the other hand, it can make the proximal wall of the main body stent 1 adhere better, and the proximal port of the main body stent 1 is sufficiently distracted, effectively reducing the risk of endo-leak. In other embodiments, the bare wave loop 11a may be completely bare, or omitted.

### Embodiment 2

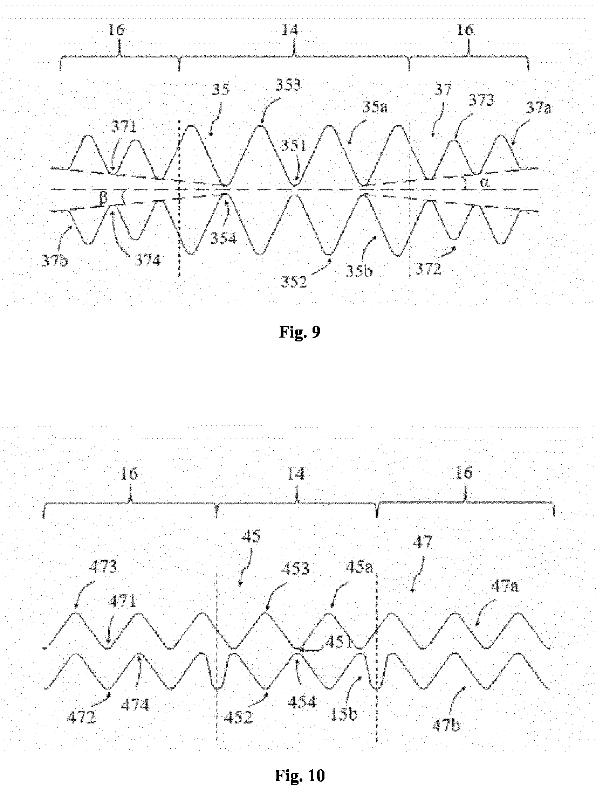

Referring to Figs. 8 and 9, the embodiment is substantially the same as the lumen stent 100 of Embodiment 1, except for the structure of the first waveform unit 35 and the second waveform unit 37 in the attachment segment 13, and how the branch stent 2 is connected to the first region 14.

As shown in Fig. 9, both the first waveform unit 35 and the second waveform unit 37 are part of the main body wave loop 11. In the embodiment, the first region 14 includes two first waveform units 35 arranged axially, a proximal first waveform unit 35a closer to the proximal end, and a distal first waveform unit 35b closer to the distal end. The second region 16 also includes two second waveform units 37, a proximal second waveform unit 37a closer to the proximal end and a distal second waveform unit 37b closer to the distal end.

The phase of the proximal first waveform unit 35a and the distal first waveform unit 35b are opposite. The phases of the proximal second waveform unit 37a and the distal second waveform unit 37b are opposite; alternatively, the proximal second waveform unit 37a and the distal second waveform unit 37b are out of phase. The wave height of the first waveform unit 35 is greater than the wave height of the second waveform unit 37. The proximal first waveform unit 35a includes a plurality of first proximal peaks 353 located substantially in the same radial plane and a plurality of first proximal valleys 351 located substantially in the same radial plane, and the distal first waveform unit 35b also includes a plurality of first distal peaks 354 located substantially in the same radial plane and a plurality of first distal valleys 352 located substantially in the same radial plane. The proximal second waveform unit 37a includes a plurality of second proximal peaks 373 located substantially in the same radial plane and a plurality of second proximal valleys 371 located in different radial planes. Distal second waveform unit 37b includes a plurality of second distal valleys 372 located substantially in the same radial plane, and a plurality of second distal peaks 374 located in different radial planes. The second proximal valley 371 circumferentially closer to the first region 14 is axially closer to the proximal end of the main body stent 1 and/or the second distal peak 374 circumferentially closer to the first region 14 is axially closer to the distal end of the main body stent 1. For example, in the embodiment, in the proximal second waveform unit 37a, the second proximal peaks 373 are each closer to the distal end than the first proximal peaks 353, the second proximal valleys 371 are each closer to the proximal end than the first proximal valleys 351, and the second proximal valleys 371 farther from the proximal first waveform unit 35a are offset proximally relative to the first proximal valleys 351 by a greater distance. As shown in Fig. 9, the line joining the second proximal valley 371 forms an angle α with the radial plane, 0 < α ≤ 20°. In other embodiments, the line connecting the second proximal valley 371 may not be straight, for example, it may be stepped. In distal second waveform unit 37b, second distal valleys 372 are each closer to the proximal end than first distal valleys 352, second distal peaks 374 are each closer to the distal end than first proximal peaks 353, and second distal peaks 374 further from distal first waveform unit 35b are offset distally a greater distance relative to first distal peaks 354. For example, the line joining the second distal peak 374 forms an angle β with the radial plane, 0 < β ≤ 20°. In other embodiments, the line joining the second distal peak 374 may not be straight, for example, it may be stepped. Since the second proximal valleys 371 are distributed on different radial planes, and the second distal peaks 374 are distributed on different radial planes when the second waveform unit 37 is radially compressed, the second proximal valleys 371 and the second distal peaks 374 do not accumulate on the same radial plane, thereby reducing the radial compression size and assembly difficulty of the lumen stent 100 while ensuring the same radial force; in addition, when the second waveform unit 37 is implanted to the slightly curved side of the aortic arch, the curved shape of the slightly curved side can be more conformed, thereby increasing the frictional force between the main body stent 1 and the inner wall of the lumen and further preventing foreshortening.

In the embodiment, the shortest axial distance (also referred to as axial spacing) between two adjacent first waveform units 35 is smaller than the shortest axial distance between two adjacent second waveform units 37. For example, the shortest axial distance between adjacent first waveform units 35 is 0 to 2 mm. In other embodiments, where the first region 14 includes more than two first waveform units 35 and the second region 16 includes more than two second waveform units 37, the shortest axial distance between any two adjacent first waveform units 35 is less than the shortest axial distance between any two adjacent second waveform units 37.

Because the shortest axial distance between two adjacent first waveform units 35 in the first region 14 is smaller than the shortest axial distance between adjacent second waveform units 37, the wave height of the first waveform unit 35 is larger than the wave height of the second waveform unit 37. Thus, the first region 14 can be shortened in the axial direction by a lesser amount than the second region 16 can be shortened in the axial direction when shortening occurs under the influence of an external force. Thus, the axial shortening rate of the first region 14 is less than the axial shortening rate of the second region 16. Even if the main body stent 1 is carried by the branch stent 2, the first region 14 greatly reduces the degree of shortening of the main body stent 1.

Referring to Fig. 8, in the embodiment, the branch tube 23 can also be connected to the first region 14 by sewing. For example, a generally axially extending tangent line may be provided between the branch tube 23 and the inner wall of the first region 14, the suture may be routed along the tangent line from the distal end of the branch tube 23 in the proximal direction (or from the proximal to the distal direction), and the suture may be terminated at a position near the proximal end of the branch tube 23 so that the branch tube 23 has a fixed segment suture-fixed with the first region 14 and located at the distal end and a movable segment movable relative to the main body stent 1 and located at the proximal end (including at least the proximal port 20a of the branch stent 2); the ratio of the length of the movable segment to the length of the fixed end is 0.1-1. This is to ensure that the movable segment can move relatively flexibly relative to the main body stent 1 and to avoid the morphological instability of the branch tube 23 under the impact of blood flow. About the transition segment 24, the side of the transition segment 24 which meets the first region 14 (such as the arcuate sheet 241) can be connected to the first region 14 via a suture, to better meet the inner wall of the transition segment 24 and the first region 14, and the gap between the transition segment 24 and the first region 14 can be minimized, to avoid blood stasis in the gap and thrombosis. In other embodiments, only one end of the transition segment 24 is connected to the window 131 (referring to Fig. 1), and the other end is connected to the branch tube 23, and regions may not be fixed to the first region 14. Further, in other embodiments, the branch stent 2 may be fixedly connected to the first region 14 by bonding or the like.

By providing the branch stent 2 with a movable proximal port 20a in the embodiment, even when the proximal port 20a of the branch stent 2 is hooked by the conveyor and pulled in the proximal direction during the withdrawal of the conveyor from the proximal end to the distal end, the proximal port 20a can move relative to the main body stent 1, so that the force of the branch stent 2 moving the main body stent 1 to the distal end can be weakened to a certain extent, thereby reducing the risk of the branch stent 2 moving the main body stent 1 to short.

The position at which the branch stent 2 is arranged and the position at which the branch stent 2 is fixed to the first region 14 also influences the anti-foreshortening performance. In the embodiment, the proximal port 20a of the branch stent 2 is substantially flush with the first proximal peak 353, or the proximal port 20a of the branch stent 2 is located between the first proximal peak 353 and the first proximal valley 351; the closest fixing point between the branch stent 2 and the first region 14 is located between the first proximal peak 353 and the first proximal valley 351. Even if the branch stent 2 of the embodiment drives the main body stent 1 to move distally, it only drives the region near the proximal first waveform unit 35a to move distally, and because the shortest axial distance between the proximal first waveform unit 35a and the distal first waveform unit 35b is small, the degree of shortening of the main body stent 1 is greatly reduced.

### Embodiment 3

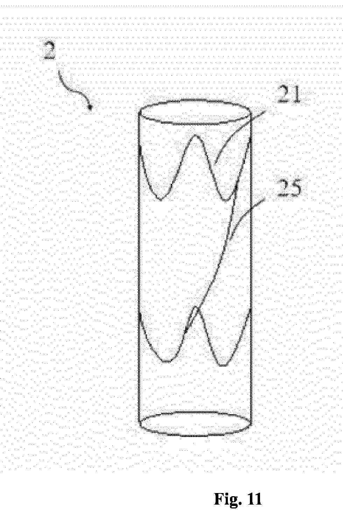

Referring to Figs. 10 and 11, the lumen stent 100 of the embodiment is substantially the same as that of the first and second embodiments 1 and 2, except for the structure of the first waveform unit 45, the second waveform unit 47, and the branch stent 2, and how the branch stent 2 is connected to the first region 14.

Referring to Fig. 10, the first proximal peak 453 of the proximal first waveform unit 45a and the second distal peak 473 of the proximal second waveform unit 47a are substantially located in the same radial plane, the first proximal valley 451 of the proximal first waveform unit 45a and the second proximal valley 471 of the proximal second waveform unit 47a are substantially located in the same radial plane, and the first distal peak 454 of the distal first waveform unit 45b and the second distal peak 474 of the distal second waveform unit 47b are substantially located in the same radial plane. The first distal valley 452 of the distal first waveform unit 45b and the second distal valley 472 of the distal second waveform unit 47b are substantially located in the same radial planevalley. That is, the wave height of the first waveform unit 45 is approximately equal to the wave height of the second waveform unit 47. Further, the proximal first waveform unit 45a and the distal first waveform unit 45b are opposite in phase. The proximal second waveform unit 47a and the distal second waveform unit 47b have the same phase, i.e. the line joining the second proximal valley 471 and the second distal valley 472 is substantially parallel to the axis of the main body stent 1, and the line joining the second proximal peak 473 and the second distal peak 474 is substantially parallel to the axis of the main body stent 1. In other embodiments, there may be a phase difference between the proximal second waveform unit 47a and the distal second waveform unit 47b.

In the embodiment, the shortest axial distance (also referred to as axial spacing) between two adjacent first waveform units 45 is smaller than the shortest axial distance between two adjacent second waveform units 47. For example, the shortest axial distance between adjacent first waveform units 45 is 1 to 2 mm. In other embodiments, where the first region 14 includes more than two first waveform units 45 and the second region 16 includes more than two second waveform units 47, the shortest axial distance between any two adjacent first waveform units 45 is less than the shortest axial distance between any two adjacent second waveform units 47.

Due to the shortest axial distance between two adjacent first waveform units 45 in the first region 14 being smaller than the shortest axial distance between adjacent second waveform units 47, the wave height of the first waveform unit 45 is substantially equal to the wave height of the second waveform unit 47. Therefore, when shortening occurs by an external force, the first region 14 can be shortened in the axial direction by a smaller amount than the second region 16 can be shortened in the axial direction, and therefore, the axial shortening rate of the first region 14 is smaller than that of the second region 16. Even if the main body stent 1 is carried by the branch stent 2, the first region 14 greatly reduces the degree of shortening of the main body stent 1. In addition, the wave height of the first waveform unit 45 is approximately equal to the wave height of the second waveform unit 47, and compared with the high-low wave design, the hollow membrane region of the attachment segment 13 (i.e. the region where the main body covering film 12 does not cover the waveform unit) of the embodiment is more uniform and rounded in the circumferential direction, and in the bent state, it is not easy to cause a large accumulation of the covering film on one side.

Referring to Fig. 11, in the embodiment, the branch tube 23 of the branch stent 2 is further provided with a support wire 25 having a linear or curved shape, and the support wire 25 may have a rigid or flexible structure, and may be made of an alloy of two or more single metals, such as cobalt, chromium, nickel, titanium, magnesium and iron, 316L stainless steel, nickel-titanium tantalum alloy, and the like, or other metal elastic materials with biocompatibility. The support wire 25 connects the most proximal branch wave loop 21 and the most distal branch wave loop 21 to prevent the branch stent 2 from shortening to some extent. In other embodiments, the support wire 25 may also extend to the transition segment 24 of the branch stent 2.

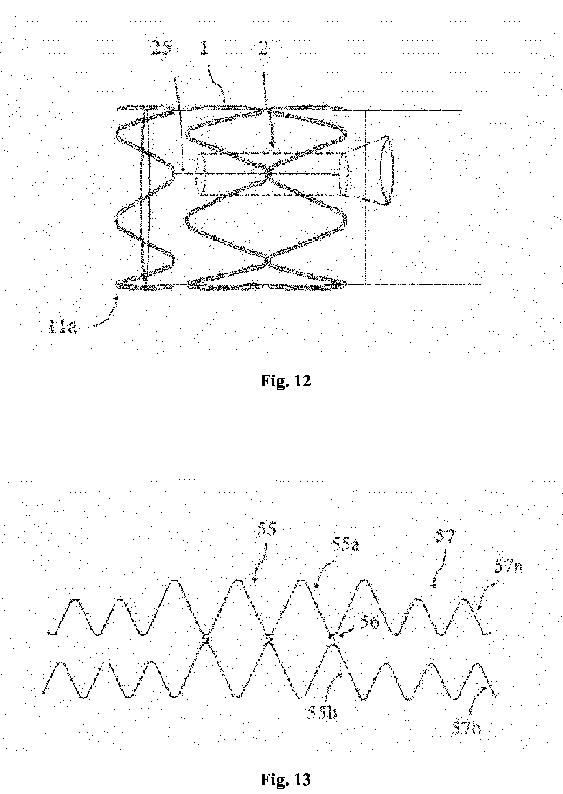

Referring to Fig. 12, in other embodiments, the support wire 25 is provided on the outer surface of the branch stent 2, and the branch stent 2 is connected to the main body stent 1 through the support wire 25. Since the support wire 25 is connected to the main body stent 1, shortening of the main body stent 1 can also be prevented to a certain extent. The proximal end of the support wire 25 may be passed over the proximal port of the branch stent 2 to improve the resistance to shortening. Further, the proximal end of the support wire 25 may extend proximally and abut the bare wave loop 11a, more effectively preventing shortening of the branch stent 2 and the main body stent 1. In other embodiments, the proximal end of the support wire 25 may also be positioned between the proximal port of the branch stent 2 and the bare wave loop 11a, without abutting the bare wave loop 11a.

### Embodiment 4

Referring to Fig. 13, the embodiment is substantially the same as the lumen stent 100 of embodiments 1 to 3, except that one or more first elastic members 56 are provided between adjacent first waveform units 55. The first elastic member 56 may be integrally woven or cut with the first waveform unit 55 or may be attached to the first waveform unit 55 by bonding, welding, or the like.

The first waveform unit 55 and the second waveform unit 57 shown in Fig. 13 are substantially the same as those of Embodiment 1. The first elastic member 56 includes a spring section having one end connected to the valley of the proximal first waveform unit 55a and the other end connected to the peak of the distal first waveform unit 55b opposite to the peak and the valley connected thereto.

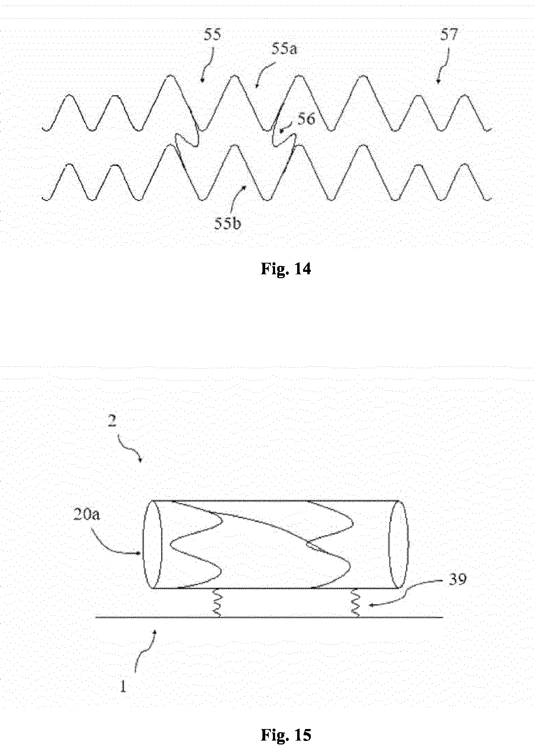

The first waveform unit 55 and the second waveform unit 57 shown in Fig. 14 are substantially the same as the first waveform unit 55 and the second waveform unit 57 of Embodiment 1, except that the phases of adjacent first waveform units 55 are the same. The first elastic member 56 is a metal wire made of a biocompatible metal elastic material including at least one wave including a Z-shape, an M-shape, a V-shape, a sinusoidal shape, and the like. The first elastic member 56 has one end connected to the rod of the proximal first waveform unit 55a and the other end connected to the rod of the distal first waveform unit 55b.

The embodiment is provided by providing a first elastic member 56, and the first elastic member 56 has a certain elasticity in the axial direction. Referring also to Fig. 1, when the branch stent 2 applies a distally moving force to the first region 14, the elasticity of the first elastic member 56 acts as a degree of cushioning, thereby effectively preventing the main body stent 1 from shortening regardless of the structure of the first waveform unit 55. Further, when the attachment segment 13 is in a radially compressed state, the first elastic member 56 may prevent the opposing peaks and valleys on the adjacent first waveform unit 55 from stacking together.

### Embodiment 5

Referring to Fig. 15, the embodiment is substantially the same as the lumen stent 100 of embodiments 1-4, except that a second elastic member 39 is further provided between the main body stent 1 and the branch stent 2 to connect the main body stent 1 and the branch stent 2. The second elastic member 39 allows at least a portion of the region of the branch stent 2 (for example, the proximal port 20a of the branch stent 2) to move relative to the main body stent 1 under the influence of an external force, deform within a certain range, and return to substantially the original position after the external force is removed. Even when the proximal port 20a of the branch stent 2 is hooked by the conveyor and pulled in the proximal direction during the withdrawal of the conveyor from the proximal end in the distal direction, the proximal port 20a can move relative to the main body stent 1, so that the force of the branch stent 2 driving the main body stent 1 to move distally can be weakened to a certain extent, thereby reducing the risk of the branch stent 2 driving the main body stent 1 to shorten. When the force of the conveyor hook is removed, the proximal port 20a of the branch stent 2 can be restored to the original position again.

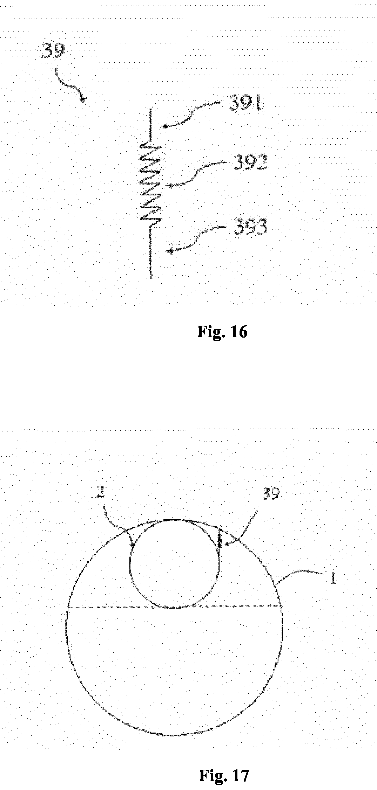

Referring also to Fig. 16, the second elastic member 39 of the embodiment has elasticity, and includes an elastic segment 392 and connecting sections (hereinafter referred to as a first connecting section 391 and a second connecting section 393 for convenience of description) provided at both ends of the elastic segment 392; the first connecting section 391 is connected to the main body stent 1, and the second connecting section 393 is connected to the branch stent 2. On the same longitudinal section, the included angle between the length extension direction of the second elastic member 39 and the outer surface of the branch stent 2 ranges from 0 to 90°, such as the included angle between the force direction of the second elastic member 39 and the outer surface of the branch stent 2 ranges from 0 to 90°.

In the embodiment, when both the main body stent 1 and the branch stent 2 are in a naturally unfolded state, the elastic segment 392 of the second elastic member 39 is in a stretched state, so that a certain tensile force is provided to the branch stent 2 via the second elastic member 39, so that the branch stent 2 abuts against the inner wall of the main body stent 1, and a certain moving space is provided for the branch stent 2 relative to the main body stent 1, thereby preventing the branch stent 2 from shaking under the impact of blood, thereby affecting the normal flow of blood. It will be appreciated that in other embodiments the second elastic member 39 may also be in a naturally relaxed state when both the main body stent 1 and the branch stent 2 are in a naturally unfolded state.

In the embodiment, the first connecting section 391 and the second connecting section 393 of the second elastic member 39 may be connected to the wave loop or the covering film of the main body stent 1 and the branch stent 2, respectively. For example, the second elastic member 39 is connected to the wave loop to prevent the covering film from being damaged by the tensile force of the second elastic member 39, thereby preventing the occurrence of an endo-leak phenomenon.

It will be appreciated that in other embodiments the second elastic member 39 may be provided with only one connecting section, in which case the other end of the elastic segment 392 is directly connected to the branch stent 2 or the main body stent 1.

For example, the second elastic member 39 may be formed of a single strand or a plurality of strands of nickel-titanium wire or stainless-steel wire in a space spiral, and the like. To ensure that the second elastic member 39 can be fully extended when subjected to recover to the original state after the force is removed and maintain certain stability, the stiffness coefficient K of the elastic segment 392 of the second elastic member 39 ranges from 0 < K <100 N/M.

The first connecting segment 391 of the second elastic member 39 can be arranged closer to the proximal end of the main body stent 1 than the second connecting segment 393 (such as the angle between the force direction of the second elastic member 39 and the outer surface of the branch stent 2 is greater than 0 degrees), so that the branch stent 2 is arranged towards the proximal end of the lumen stent 100 under the tensile force of the second elastic member 39, and thus the branch stent 2 is sufficiently close to the inner surface of the main body stent 1, to reduce or eliminate the gap between the main body stent 1 and the branch stent 2, and thus minimize the influence of the branch stent 2 on the blood flow in the main body stent 1 and reduce the formation of thrombus.

As shown in Figs. 15 and 16, the second elastic member 39 is provided between the outer surface of the branch stent 2 away from the central axis of the main body stent 1 and the main body stent 1, thereby facilitating the connection of the branch stent 2 and the main body stent 1, fitting the branch stent 2 to the inner surface of the main body stent 1 and reducing the deformation of the branch stent 2, while avoiding the influence of the blood flow in the lumen of the main body stent 1 due to the large spatial distribution of the second elastic member 39 in the lumen of the main body stent 1. Accordingly, the first connecting segment 391 connected to the inner surface of the main body stent 1 may be distributed in a circumferential region corresponding to a horizontal plane tangent to the lower edge of the proximal end surface of the branch stent 2; a horizontal plane tangent to the lower edge of the proximal end surface of the branch stent 2 is shown by a broken line in Fig. 17.

The number of the second elastic members 39 of the embodiment is two, and the second elastic members 39 are disposed on the same side of the branch stent 2 in the axial direction. In other examples of the embodiment, one or more second elastic members 39 may also be provided, for example, a plurality of second elastic members 39 may be axially provided on the same side of the branch stent 2, and the plurality of second elastic members 39 may be axially arranged or offset. From the proximal end toward the distal end of the branch tube 23, the deformable length of the plurality of second elastic members 39 can be gradually reduced so that the proximal end region of the branch tube 23 can be moved relative to the main body stent 1 to a greater extent. In other embodiments, one or more second elastic members 39 may also be provided on the transition segment 24.

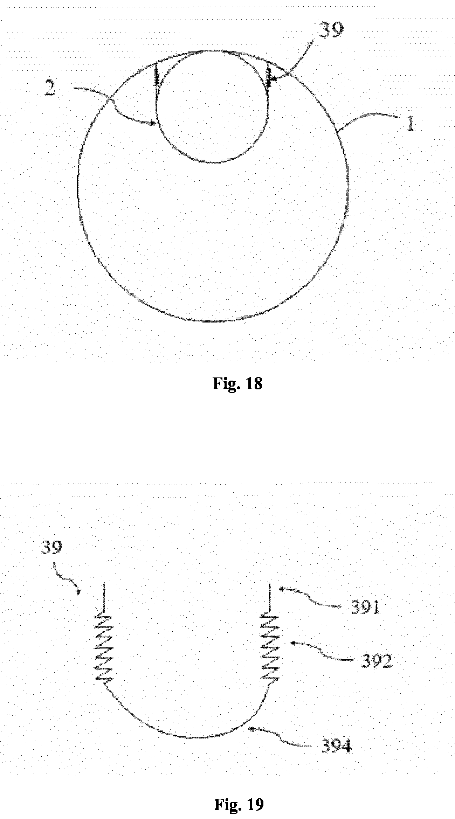

Referring to Fig. 18, in other embodiments, second elastic members 39 are simultaneously provided on both side surfaces of the branch stent 2, and the main body stent 1 and the branch stent 2 are simultaneously connected by the second elastic members 39 on both sides so that the stress of the branch stent 2 is more balanced and the stability of the branch stent 2 is further improved.

Referring to Fig. 19, in other embodiments, the second elastic member 39 includes a middle section 394 and elastic segments 392 provided at both ends of the middle section 394. The middle section 394 has an arc shape and is connected to the branch stent 2, such that the middle section 394 is connected to the outer surface of the branch stent 2 facing the central axis of the main body stent 1, and the middle section 394 may conform to the shape of a part of the outer surface of the branch stent 2, so that the middle section 394 completely conforms to the outer surface of the branch stent 2 facing the central axis of the main body stent 1, or may have another shape and only partially conforms to the outer surface of the branch stent 2 facing the central axis of the main body stent 1. The elastic segments 392 at both ends of the middle section 394 are provided on both sides of the branch stent 2, respectively, and the elastic segments 392 at both sides are connected to the main body stent 1 via the first connecting section 391, so that the second elastic member 39 forms a U-shaped structure, thereby expanding the contact area with the second elastic member 39 and the branch stent 2, so that the tensile force provided by the second elastic member 39 to the branch stent 2 is more evenly distributed, reducing the damage to the covering film of the branch stent 2, and further improving the deformation resistance and deformation recovery ability of the branch stent 2. The elastic segment 392 may be crimped or welded or integrally formed with the intermediate section 394 via the second connecting section 393.

In other embodiments, the intermediate section 394 is part of the structure of the branch stent 2, i.e. the intermediate section 394 is one of a plurality of branch wave loops 21 (cf. Fig. 6) constituting the branch stent 2, and the wave loops are arranged to be disconnected, and an elastic segment 392 is arranged at the disconnection of both ends of the wave loops. In other embodiments, the intermediate section 394 may be connected to the main body stent 1, or the intermediate section 394 may be part of the structure of the main body stent 1, and the constituent structure is consistent with the relationship between the intermediate section 394 and the branch stent 2, which will not be described in detail herein.

### Embodiment 6

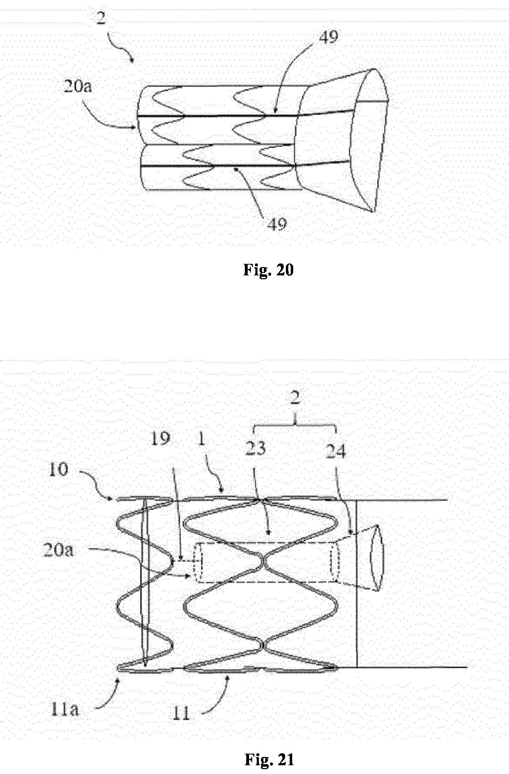

Referring to Fig. 20, the embodiment is substantially identical to the lumen stent 100 of embodiments 1-5, except for the structure and arrangement position of the second elastic member 49.

The second elastic member 49 of the embodiment is attached to the inner and/or outer surface of the branch stent 2, and one end of the second elastic member 49 is closer to the proximal end of the branch stent 2 than the other end. The second elastic member 49 serves to provide elastic force for restoring deformation to the branch stent 2.

The second elastic member 49 of the embodiment includes a support rod, and the material of the support rod can be super-elastic nickel-titanium alloy and has a diameter of 0.05 mm to 0.3 mm. In the embodiment, the second elastic member 49 and the branch stent 2 are of a split-type structure, and for example, the proximal end of the second elastic member 49 is connected to the proximal end of the branch stent 2, and the distal end of the second elastic member 49 is connected to the distal end of the branch stent 2, to provide a supporting force for the branch stent 2 in the axial direction, to improve the ability of the branch stent 2 to recover deformation in the axial direction, and at the same time, allow at least part of the region of the branch stent 2 (including the proximal port 20a) to move relative to the main body stent 1 under the action of an external force, to deform within a certain range, and recover substantially to the initial position after the external force is removed. The particular shape of the support rod is not required and maybe one or a combination of linear, wavy, or arcuate.

When a ring-shaped supporting member is provided at the window 131 (refer to Fig. 1), the distal end of the second elastic member 49 is connected to the supporting member; when the branch stent 2 is subjected to the force of the conveyor since the second elastic member 49 is connected to the main body stent 1, the shape of the branch stent 2 is more stable, and the branch stent 2 can recover to the original state faster after deformation.

In other embodiments, the second elastic member 49 may also be part of the structure of the branch stent 2. For example, the second elastic member 49 is of an integral structure with the branch tube 23, the proximal end of the second elastic member 49 is provided at the proximal end of the branch tube 23, the distal end of the second elastic member 49 extends to the distal end of the transition segment 24, and is connected to the transition segment 24 using sewing, crimping, bonding, and the like to realize the arrangement of the second elastic member 49 along the axial direction of the branch stent 2, to provide a supporting force for the branch stent 2 and an elastic force for restoring deformation.

In other embodiments, the second elastic member 49 may be provided only at the proximal end region of the branch tube 23, for example, one end of the second elastic member 49 is provided at the proximal end portion of the branch tube 23 and the other end is provided between the proximal and distal end portions of the branch tube 23. The region of the branch tube 23 where the second elastic member 49 is not provided may be fixedly connected to the first region 14 by sewing, bonding, and the like. The ratio of the axial length of the region on the branch tube 23 where the second elastic member 49 is provided to the total length of the branch tube 23 is 0.2 to 0.5, which is advantageous in reducing the radial compression size of the lumen stent 100.

### Embodiment 7

Referring to Fig. 21, the embodiment is substantially the same as the lumen stent 100 of embodiments 1-4, except that the lumen stent 100 of the embodiment further includes a connector 19, and the main body stent 1 includes an anchoring portion 10.

In the embodiment, the anchoring portion 10 is provided at the proximal end of the main body stent 1, and when the lumen stent 100 is implanted into the lumen of a human body, an anchor forms between the anchoring portion 10 and the inner wall of the lumen of the human body, to prevent the main body stent 1 from shifting intraoperatively and long-term to a certain extent.

In the embodiment, the anchoring portion 10 includes one or more bare wave loops 11a provided at the proximal end of the main body stent 1; the bare wave loops 11a are fixedly connected to the proximal end of the main body covering film 12, and at least part of the bare wave loops 11a are exposed, such as the main body covering film 12 only partially covers the bare wave loops 11a or does not cover the bare wave loops 11a.

In other embodiments, the anchoring portion 10 may further include one or more anchors (not shown) provided on the bare wave loop 11a, for example, a plurality of peaks of the bare wave loop 11a extend proximally with anchor posts, the anchor post is provided with an anchor pin which integrally formed with the anchor post, and the anchor pin extends outwardly from the anchor post on which located and point distally. After the lumen stent 100 is implanted into the lumen of the human body, the anchor pin can form a reliable fixed connection between the proximal end of the main body stent 1 and the inner wall of the lumen of the human body, further improving the anchoring force between the anchoring portion 10 and the inner wall of the lumen of the human body. It will be appreciated that in other embodiments, the anchoring portion 10 may omit the bare wave loop 11a and the anchor pins in the anchoring portion 10 may be attached directly to the proximal edge of the main body stent 1, again providing an anchoring effect.

In the embodiment, the radial support force of the bare wave loop 11a is greater than the radial support force of the main body wave loop 11. For example, the ratio of the radial supporting force of the single bare wave loop 11a to the radial supporting force of the single main body wave loop 11 ranges from 1.1 to 2. The radial support force of the bare wave loop 11a and the main body wave loop 11 can be adjusted by adjusting the material, wire diameter, wave angle, wave height, wave number, and other parameters of the bare wave loop 11a and the main body wave loop 11. For example, when other parameters of the bare wave loop 11a and the main body wave loop 11 are the same, the material stiffness of the bare wave loop 11a can be made greater than that of the material used for the main body wave loop 11, so that the radial support force of the bare wave loop 11a is greater than that of the main body wave loop 11; when the bare wave loop 11a and the main body wave loop 11 are made of the same material, the other waveform parameters can be kept the same, and the wire diameter of the bare wave loop 11a is greater than the wire diameter of the main body wave loop 11, so that the radial support force of the bare wave loop 11a is greater than the radial support force of the main body wave loop 11.

Because the bare wave loop 11a is at least partially exposed, and the radial support force of the bare wave loop 11a is greater, after the lumen stent 100 is implanted in the lumen of a human body, the bare wave loop 11a may have a better anchoring capability, i.e. a better capability of resisting relative axial displacement.

In the embodiment, the free end 20a of the branch tube 23 is movable relative to the main body stent 1. For example, the branch tube 23 and the inner wall of the main body stent 1 have a tangent line extending in a substantially axial direction, the suture can be routed along the tangent line from the distal end of the branch tube 23 in a proximal direction (or from the proximal end to the distal direction), and the suture can be ended at a position close to the proximal end of the branch tube 23 so that the branch tube 23 has a fixed segment suture-fixed with the main body stent 1 and located at the distal end and a movable segment movable relative to the main body stent 1 and located at the proximal end (at least including the free end 20a of the branch stent 2); the ratio of the length of the movable segment to the length of the fixed end is 0.1 to 1; this is to ensure that the movable segment can move relatively flexibly relative to the main body stent 1, and also to avoid the morphological instability of the branch tube 23 under the impact of blood flow. About the transition segment 24, the side of the transition segment 24 which is fitted with the main body stent 1 can be connected with the main body stent 1 via a suture, so that the transition segment 24 can be better fitted with the inner wall of the main body stent 1, and the gap between the transition segment 24 and the main body stent 1 can be minimized, to avoid blood stasis in the gap, thus causing thrombosis. In other embodiments, only one end of the transition segment 24 is connected to the window 131 (refer to Fig. 1), the other end is connected to the branch tube 23, and other regions may not be fixed to the main body stent 1. In addition, in other embodiments, the branch stent 2 may be fixedly connected to the main body stent 1 by bonding, heat-fusing, or the like.

The free end 20a of the branch tube 23 is connected to the anchoring portion 10 via the connector 19. In the embodiment, the connector 19 includes a support rod, one end of which is connected to the anchoring portion 10 (for example, the bare wave loop 11a of the anchoring portion 10) and the other end of which is connected to the free end 20a of the branch stent. For example, one end of the support rod may be fixedly connected to the anchoring portion 10 by welding, bonding, heat-fusing, sewing, winding, and the like and the other end of the support rod may be fixedly connected to one or more of the branch wave loop 21 at the proximal end of the branch tube 23, the branch covering film 22 at the free end 20a, and the second annular support member.

The material of the above-mentioned support rod can be a biocompatible polymer material, a metal material, and the like; the diameter of the support rod is 0.05 mm to 0.3 mm, and the specific shape of the support rod is not required and can be straight and/or curved.

In the embodiment, the support rod is integrally attached to the surface of the main body stent 1, and the support rod can move relative to the main body covering film 12, and the support rod can move at least part of the region of the branch stent 2 (for example, the free end 20a of the branch stent 2) relative to the main body stent 1 under the action of an external force, can be deformed within a certain range, and can be returned to substantially the original position after the external force is removed. Even when the free end 20a of the branch stent 2 is hooked by the conveyor and pulled in the proximal direction during withdrawal of the conveyor from the proximal to the distal direction, the free end 20a can move relative to the main body stent 1, and the free end 20a is connected to the anchoring portion 10 via the connector 19, so that the force of the conveyor hooking the branch stent 2 is transmitted to the anchoring portion 10 with stronger anchoring ability along the connector 19 without shortening or shifting the main body stent 1 due to the direct action on the main body covering film 12. When the force with which the conveyor is hooked is removed, the free end 20a of the branch stent 2 can again return to the original position.

In other embodiments, the support rod may be articulatable with respect to the anchoring portion 10 and/or the free end 20a. For example, an articulating portion may be provided at an end of the support rod, the articulating portion including a collar and/or a wrap (for example, the wrap including a helically wound filament), the articulating portion being articulating with the anchoring portion 10 and/or the free end 20a such that the support rod is moveable in a lengthwise or circumferential direction relative to the anchoring portion 10 and/or the free end 20a. The advantage of this arrangement is, at least, that the distance between the main body stent 1 and the free ends 20a of branch stent 2 may vary when the lumen stent 100 is in a radially compressed state or when the lumen stent 100 is subjected to a blood impact since the movably connected support rods may be moved to some extent in the length direction thereof, thereby preventing the support rods from restraining the variation in the distance between the main body stent 1 and the branch stent 2, thereby preventing the main body stent 1 or the branch stent 2 from being damaged by pulling the main body stent 1 or the branch stent 2 in a radially compressed state or is subjected to a blood impact. In addition, the support rod movably connected to the anchoring portion 10 and/or the free end 20a allows the free end 20a to move more flexibly relative to the main body stent 1, and the conveyor can be disengaged from the free end 20a when the free end 20a is bent to a certain degree.

### Embodiment 8

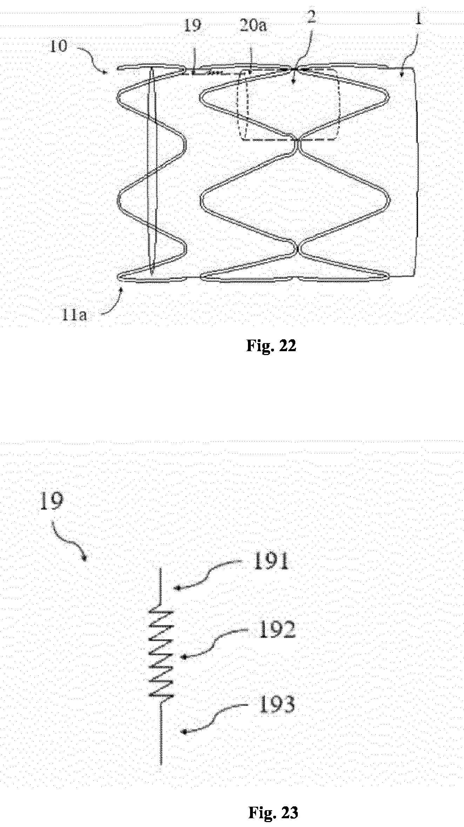

Referring to Figs. 22 and 23, the embodiment is substantially the same as the lumen stent 100 of embodiment 7, except that the connector 19 of the embodiment has elasticity, and includes an elastic segment 192 and connecting segments (hereinafter, referred to as a first connecting segment 191 and a second connecting segment 193 for convenience of description) provided at both ends of the elastic segment 192; the first connecting segment 191 is connected to the main body stent 1 and the second connecting segment 193 is connected to the branch stent 2. On the same longitudinal section, the included angle between the length extension direction of the connector 19 and the outer surface of the branch stent 2 is in the range of 0 to 60°, such as the included angle between the force direction of the connector 19 and the outer surface of the branch stent 2 is in the range of 0 to 60°.

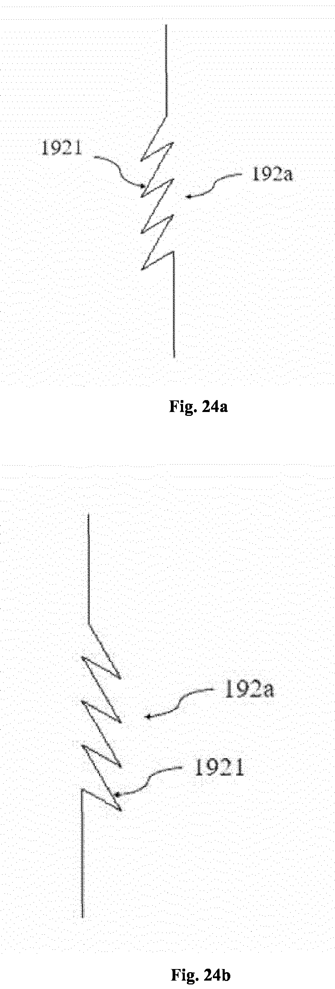

The elastic segment 192 includes one or more of a helical spring structure, an elastomer member, and a planar folding structure. The helical spring structure includes one or more of an extension spring, a progressive spring, and a linear spring. The elastomer member includes a member made of a material having elasticity, for example, an elastic cord made of an elastic polymer material, an elastic wire, or an elastic rod made of a super-elastic metal material such as nickel-titanium alloy, and the like. The planar folding structure may be elongated and shortened along its length, including one or more of Z-waves, M-waves, V-waves, and sine waves. As shown in Figs. 24a and 24b, to further reduce the radial compression dimension of the lumen stent 100, the planar folding structure includes a sawtooth wave 192a, the sawtooth wave 192a including one or more sawtooths1921 inclined toward the anchoring portion 10 or toward the free end 20a of the branch stent 2, which such sawtooth wave 192a may be folded to some extent in the lateral direction (the direction perpendicular to the length direction), thereby reducing the radial compression dimension of the lumen stent 100.

In the embodiment, when both the main body stent 1 and the branch stent 2 are in a naturally unfolded state, the elastic segment 192 of the connector 19 is in a stretched state, so that a certain tensile force is provided to the branch stent 2 via the connector 19, so that the branch stent 2 abuts against the inner wall of the main body stent 1, and a certain moving space is provided for the branch stent 2 relative to the main body stent 1, thereby preventing the branch stent 2 from shaking under the impact of blood, affecting the normal flow of blood. It will be appreciated that in other embodiments when both the main body stent 1 and the branch stent 2 are in a naturally unfolded state, connector 19 may also be in a naturally relaxed state.

In the embodiment, the first connection segment 191 and the second connection segment 193 of the connector 19 may be connected to one or more of the anchoring portion 10, the branch wave loop 21 of the branch stent 2, the branch covering film 22 at the free end 20a (refer to Fig. 6), and the second annular support. For example, the connector 19 is connected to the branch wave loop 21 or the second annular support member to prevent the branch covering film 22 from being damaged by the tensile force of the connector 19, thereby preventing the occurrence of an endo-leak phenomenon.

It will be appreciated that in other embodiments the connector 19 may be provided with only one connecting section, in which case the other end of the elastic segment 192 is directly connected to the branch stent 2 or the main body stent 1.

For example, the connector 19 may be formed of a single strand or a plurality of strands of nickel titanium wire or a stainless-steel wire spiral, and the like. To ensure that the connector 19 can be fully extended when subjected to a suitable pulling force, and can recover to the original state and maintain a certain stability after the force is removed, the range value of the stiffness coefficient K of the elastic segment 192 of the connector 19 is 0 < K <100 N/M.

The first connecting segment 191 of the connector 19 may be arranged closer to the anchoring portion 10 than the second connecting segment 193 (such as the angle between the force direction of the connector 19 and the outer surface of the branch stent 2 is greater than 0 degrees), so that the branch stent 2 is arranged towards the anchoring portion 10 under the tensile force of the connector 19, thereby making the branch stent 2 sufficiently close to the inner surface of the main body stent 1, thereby reducing or eliminating the gap between the main body stent 1 and the branch stent 2, thereby minimizing the influence of the branch stent 2 on the blood flow in the main body stent 1 and reducing the formation of thrombi.

In other embodiments, the first connection segment 191 and the second connection segment 193 may be omitted, and both ends of the elastic segment 192 are connected to the anchoring portion 10 and the free end 20a of the branch stent 2, respectively.

The connector of the embodiment has elasticity so that the free end 20a of the branch stent 2 can move more flexibly relative to the main body stent 1, even if the free end 20a of the branch stent 2 is hooked and pulled in the proximal direction by the conveyor, the free end 20a can be bent more easily, and when bent to a certain degree, the conveyor can be separated from the free end 20a. In addition, when the lumen stent 100 is in a radially compressed state or when the lumen stent 100 is subjected to a blood impact, the connector having elasticity can accommodate a change in the distance between the main body stent 1 and the free ends 20a of branch stent 2, and prevent damage to the main body stent 1 or the branch stent 2 due to excessive pulling of the main body stent 1 or the branch stent 2 in the radially compressed state or the blood impact.

### Embodiment 9

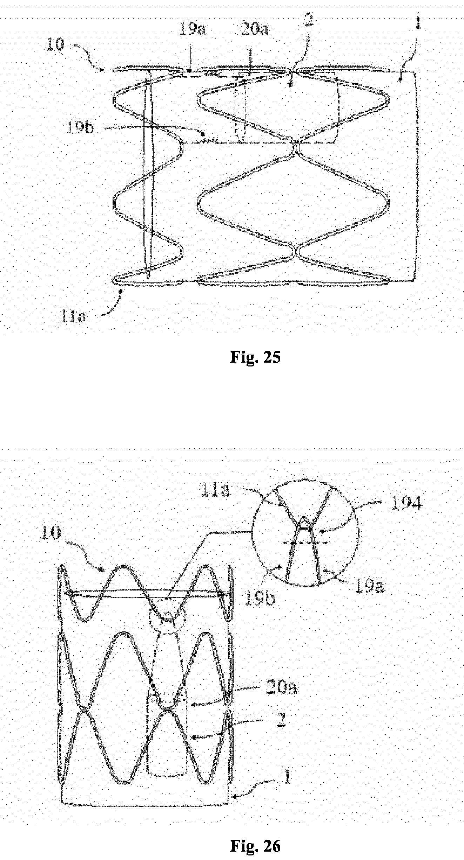

Referring to Fig. 25, the embodiment is substantially the same as the lumen stent 100 of embodiment 8, except that the lumen stent 100 of the embodiment includes a first connector 19a and a second connector 19b. For example, a first connector 19a connects the first side of the branch stent 2 (a side facing the inner wall of the main body stent 1 and close to the inner wall of the main body stent 1), and a second connector 19b connects a second side of the branch stent 2 (a side facing the central axis of the main body stent 1 and close to the central axis of the main body stent 1). Connecting the first side and the second side of the branch stent 2 with the first connector 19a and the second connector 19b, respectively, makes the stress of the branch stent 2 more balanced, further improving the stability of the branch stent 2. Further, the rigidity of the first connector 19a and the second connector 19b may be different. For example, if the conveyor is more likely to hook on the second side, the stiffness (for example, axial stiffness, i.e. the ability to resist deformation along the length) of the second connector 19b connected to the second side may be made less than the stiffness of the first connector 19a connected to the first side, and if the conveyor is more likely to hook on the first side, the stiffness of the first connector 19a connected to the first side may be made less than the stiffness of the second connector 19b connected to the second side. This arrangement facilitates a more flexible movement of the free end 20a of the branch stent 2. In other embodiments, both connectors 19 may be provided on the first side or both on the second side. In other embodiments, more than two connectors 19 may be provided.

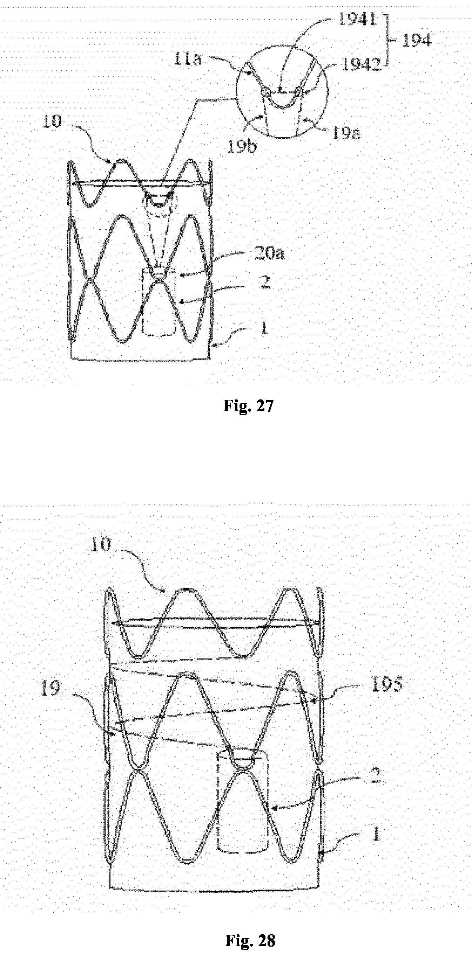

Referring to Fig. 26, in other embodiments, an intermediate member 194 is provided between the first connector 19a and second connector 19b, and the intermediate member 194 is connected to the proximal end portions of the first connector 19a and second connector 19b and is connected to the bare wave loop 11a. In the embodiment, the intermediate member 194 is movably connected to the valley of the bare wave loop 11a. For example, the intermediate member 194 has an arc shape and is movably connected to a bare wave loop 11a (such as a valley of the bare wave loop 11a) by a hooking connection or a winding connection, and the like; or, with reference to Fig. 27, the intermediate member 194 includes a limiting member 1941 (such as a long strip-shaped filament or a rod with elasticity); the limiting member 1941 is provided with at least one sliding connector 1942 (such as a ring or a tube, and the like), the sliding connector 1942 is sheathed on the rod of the bare wave loop 11a and can slide along the length direction of the rod of the bare wave loop 11a, the limiting member 1941 is used for limiting the sliding range of the sliding connector 1942, for example, both ends of the long strip-shaped limiting member 1941 are provided with the sliding connectors 1942, and the two sliding connectors 1942 are connected to the proximal ends of the two connectors 19, respectively; alternatively, one end of the elongated limiting member 1941 is fixedly connected to the bare wave loop 11a, and the other end is provided with a sliding connector 1942; the proximal end of the first connector 19a is connected to the sliding connector 1942, and the proximal end of the second connector 19b is connected to the limiting member 1941 or the bare wave loop 11a. The function of the sliding connection between the intermediate member 194 and the bare wave loop 11a is that when one side of the branch stent 2 where one of the connectors is located is hooked by the conveyor, the intermediate member 194 can move relative to the anchoring portion 10, so that the change in the distance between the proximal end of the connector and the anchoring portion 10 can be accommodated to a certain extent, so that the proximal port 20a of the branch stent 2 can move relative to the main body stent 1 more flexibly, and after the conveyor and the branch stent 2 fall off, the intermediate member 194 can bring the connector back to the initial position. In other embodiments, the intermediate member 194 may be fixedly attached to the valley of the bare wave loop 11a.

### Embodiment 10

The embodiment is substantially the same as the embodiment 7, except that, with reference to Fig. 28, the connector 19 includes a helical segment 195; the helical segment 195 is integrally spirally extended around the central axis of the main body stent 1 and the helical segment 195 at least partially conforms to the inner surface of the main body stent 1 and is movable relative to the inner surface of the main body stent 1, for example, only two ends of the connector 19 are connected to the anchoring portion 10 and the branch stent 2, respectively, and the remaining area including the helical segment 195 merely conforms to (or abuts against) the inner surface of the main body stent 1 without a connection point with the main body stent 1.

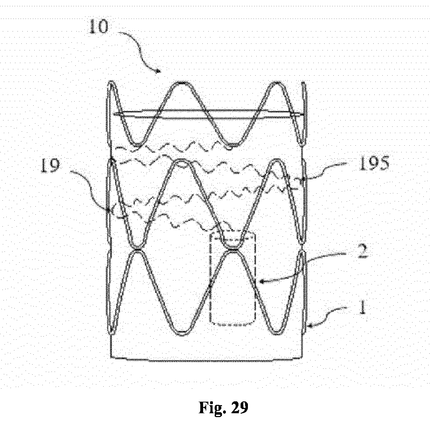

The helical segment 195 may extend helically one or more turns around the central axis of the main body stent 1, and in other embodiments, the helical segment 195 may extend helically less than one turn around the central axis of the main body stent 1. Since the helical segment 195 itself has a spiral shape, and the helical structure itself has an elastic recovery ability (such as the ability to deform under the action of an external force and recover the original shape after removing the external force), it is not required that the material for manufacturing the helical segment 195 has good elasticity, and can be made of various types of biocompatible materials, such as high molecular materials or metal materials. Referring to Fig. 29, to further improve the elasticity of the helical segment 195, the helical segment 195 further includes a helical structure formed by spiral winding of one or more of a helical spring structure including one or more of an extension spring, a progressive spring, and a linear spring, a planar folding structure, and an elastomer member, and the helical segment 195 includes a helical structure formed by spiral winding of one or more of the above-mentioned springs. The elastomer member includes a member made of a material having elasticity, for example, the helical segment 195 includes an elastic rope made of an elastic polymer material, a helical structure formed by spirally surrounding an elastic wire or an elastic rod made of a super-elastic metal material (such as nickel-titanium alloy). The planar folding structure includes one or more of Z-shaped wave, M-shaped wave, V-shaped wave, and sine wave, and the planar folding structure of the embodiment can be radially compressed and has a radial expansion capability, can achieve radial contraction under the action of an external force, and can self-expand or recover to and maintain the original shape by mechanical expansion after the external force is removed. The helical segment 195 includes a helical structure formed by spirally winding the above-described planar folding structure.

The helical segment 195 of the embodiment has an elastic recovery capability to allow more flexible movement of the free end 20a of the branch stent 2 relative to the main body stent 1. In addition, when the lumen stent 100 is in a radially compressed state or when the lumen stent 100 is subjected to a blood impact, the connector having the helical segment 195 can also accommodate a change in the distance between the free ends 20a of the main body stent 1 and the branch stent 2 to prevent damage to the main body stent 1 or the branch stent 2 due to excessive pulling of the main body stent 1 or the branch stent 2 in the radially compressed state or the blood impact. Further, since the helical segment 195 is at least partially attached to the inner surface of the main body stent 1, the adherence, displacement resistance, and shortening resistance of the main body stent 1 in the area where the helical segment 195 is located can be increased.

Further, the connectors of the above-mentioned embodiments 7 to 10 can also be made of biodegradable materials, for example, degradable polymers such as degradable polyesters and/or degradable polyanhydrides can be used, and degradable metal materials such as magnesium-based, iron-based, and zinc-based alloys can also be used. The advantage of this arrangement is, at least, that it avoids the problems of late restenosis, late thrombosis, and the like in the blood vessel, which contributes to improved biological safety.

As will be appreciated by those skilled in the art, the anchoring portion 10 of the above-described embodiments 7 to 10 is located at the proximal end of the main body stent 1, and the free end 20a of the branch stent 2 is located at the proximal end of the branch stent 2. In other embodiments, the anchoring portion 10 may be located at the distal end of the main body stent 1 and the free end 20a of the branch stent 2 at the distal end of the branch stent 2. The embodiments are not limited to the specific positions of the anchoring portion 10 and the free end 20 of the branch stent 2 as long as the effects of the embodiments can be achieved.

As will be appreciated by those skilled in the art, the attachment segment 13 in the above-described embodiments 7 to 10 may be different from the attachment segment 13 described in embodiments 1-4, and in embodiments 7 to 10, whether or not the attachment segment 13 includes a first region 14 and a second region 16 connected to the first region 14 in the circumferential direction, whether or not the axial shortening rate of the first region 14 is smaller than the axial shortening rate of the second region 16, without affecting the above-described joint function of the connector 19 and the anchoring portion 10 alone. In the lumen stent 100 of embodiments 7 to 10, even if the free end 20a of the branch stent 2 is hooked by the stent and pulled in the proximal direction during the withdrawal of the stent from the proximal end in the distal direction, the free end 20a can move relative to the main body stent 1, and the free end 20a is connected to the anchoring portion 10 via the connector 19 so that the force of the stent hooking the branch stent 2 is transmitted to the anchoring portion 10 with stronger anchoring ability along the connector 19 without the main body stent 1 being shortened or displaced by directly acting on the main body covering film 12 or the main body wave loop 11.

Although embodiments have been provided for illustrative purposes, those skilled in the art will appreciate that various modifications, additions, and substitutions are possible, without departing from the scope and spirit of the embodiments.

## Claims

1. A lumen stent, comprising:
a main body stent and a branch stent, wherein the main body stent comprises a tubular attachment segment, the attachment segment comprising a first region and a second region connected to the first region in a circumferential direction;
the branch stent is provided in a lumen of the attachment segment and connected to the first region; and
an axial shortening rate of the first region is smaller than an axial shortening rate of the second region.

2. The lumen stent according to claim 1, wherein the first region comprises a plurality of first waveform units arranged axially, and the second region comprises a plurality of second waveform units arranged axially, a shortest axial distance between two adjacent first waveform units being less than a shortest axial distance between two adjacent second waveform units.

3. The lumen stent according to claim 2, wherein a wave height of the first waveform unit is greater than a wave height of the second waveform unit.

4. The lumen stent according to claim 2, wherein the first region comprises adjacent proximal first waveform units and distal first waveform units;
the proximal first waveform units comprise first proximal valleys, and the distal first waveform units comprise first distal peaks; and
at least one of the first proximal valleys is opposite to one of the first distal peaks, and a line between the first distal peak and the first proximal valley is substantially parallel to an axis of the main body stent.

5. The lumen stent according to claim 2, wherein the second region comprises a proximal second waveform unit and a distal second waveform unit;
the proximal second waveform unit comprises a plurality of second proximal valleys located in different radial planes, and the distal second waveform unit comprises a plurality of second distal peaks located in different radial planes; and
the second proximal valley circumferentially closer to the first region is closer to a proximal end of the main body stent, and/or the second distal peak circumferentially closer to the first region is closer to a distal end of the main body stent.

6. The lumen stent according to claim 2, wherein a wave height of the first waveform unit is substantially equal to a wave height of the second waveform unit;
phases of adjacent first waveform units are opposite, and
phases of adjacent second waveform units are the same or have a phase difference.

7. The lumen stent according to any one of claims 1 to 6, wherein the first region comprises a plurality of first waveform units arranged axially, and at least one first elastic member is provided between adjacent first waveform units.

8. The lumen stent according to any one of claims 1 to 6, wherein the branch stent has a proximal port movable relative to the main body stent.

9. The lumen stent according to claim 8, further comprising:
a second elastic member, wherein the second elastic member is disposed between an inner surface of the main body stent and an inner surface of the branch stent; and
the second elastic member is elastic and is used to limit a range of motion of the proximal port of the branch stent.

10. The lumen stent according to claim 1, wherein at least one of the branch stents is provided with a linear or curved support wire, one end of the support wire being closer to a proximal end of the branch stent than the other end.

11. The lumen stent according to claim 10, wherein the support wire is located on an outer surface or inner surface of the branch stent.

12. The lumen stent according to claim 11, wherein a proximal end of the support wire passes over a proximal port of the branch stent.

13. The lumen stent according to claim 12, wherein the main body stent comprises a main body covering film and at least one main body wave loop disposed within the main body covering film; and
the proximal end of the support wire and the main body wave loop abut each other when the lumen stent is in a naturally unfolded state.

14. A lumen stent, comprising:
a tubular main body stent, comprising an anchoring portion;
a branch stent provided within a lumen of the main body stent and comprises a free end movable relative to the main body stent; and
a connector, that connects the free end of the branch stent and the anchoring portion.

15. The lumen stent according to claim 14, wherein the main body stent comprises a covering film and a plurality of axially spaced main body wave loops connected to the covering film; and the anchoring portion comprises a bare wave loop and/or an anchor, a radial support force of the bare wave loop being greater than a radial support force of the main body wave loop.

16. The lumen stent according to claim 14, wherein the free end of the branch stent is located at a proximal end of the branch stent, and the anchoring portion is located at a proximal end of the main body stent; or
the free end of the branch stent is located at a distal end of the branch stent, and the anchoring portion is located at a distal end of the main body stent.

17. The lumen stent according to any one of claims 14 to 16, wherein the connector comprises a linear or curved support rod; and
one end of the support rod is connected to the anchoring portion, and the other end of the support rod is connected to the free end of the branch stent.

18. The lumen stent according to any one of claims 14 to 16, wherein the connector comprises an elastic segment, two ends of the elastic segment connecting to the anchoring portion and the free end of the branch stent, respectively; and
the elastic segment comprises one or more of a helical spring structure, a planar folding structure, and an elastomer member.

19. The lumen stent according to claim 18, wherein the planar folding structure comprises a sawtooth wave; and
the sawtooth wave comprises a sawtooth tilted toward the anchoring portion or the branch stent.

20. The lumen stent according to any one of claims 14 to 16, wherein the connector comprises a helical segment; the helical segment extends helically entirely around an axis of the main body stent; and the helical segment at least partially conforms to and is movable relative to an inner surface of the main body stent.

21. The lumen stent according to claim 20, wherein the helical segment comprises a helical structure formed by a helical wrapping of one or more of a helical spring structure, a planar folding structure, and an elastomer member.

22. The lumen stent according to any one of claims 14 to 16, wherein the connector is fixedly or movably connected to the anchoring portion, and the connector is fixedly or movably connected to the free end of the branch stent.

23. The lumen stent according to any one of claims 14 to 16, wherein the lumen stent comprises two connectors, and the two connectors are provided on two sides of the branch stent, respectively.

24. The lumen stent according to claim 23, further comprising an intermediate member; the intermediate member is movably connected to the anchoring portion, and the intermediate member is connected to at least one connector.

25. The lumen stent according to claim 23, wherein the lumen stent comprises a first connector and a second connector; the second connector is closer to an axis of the main body stent than the first connector, and stiffness of the second connector is less than stiffness of the first connector.
